# EUROPÄISCHE PATENTSCHRIFT

(11) **EP 1 539 299 B1**
(45) Veröffentlichungstag und Bekanntmachung des Hinweises auf die Patenterteilung: **17.08.2016**
(21) Anmeldenummer: 03797137.1
(22) Anmeldetag: 15.09.2003
(51) Int. Cl.: A61N 5/10

(54) **ANORDNUNG ZUR DURCHF HRUNG EINER PROTONENTHERAPIE**
SYSTEM FOR PERFORMING PROTON THERAPY
INSTALLATION POUR REALISER UNE THERAPIE PROTONIQUE

(30) Priorität: 18.09.2002 CH 157902
(43) Veröffentlichungstag der Anmeldung: 15.06.2005
(73) Patentinhaber: PAUL SCHERRER INSTITUT, 5232 Villigen PSI (CH)
(72) Erfinder: PEDRONI, Eros, CH-5200 Brugg (CH)
(74) Vertreter: Fischer, Michael
(86) Internationale Anmeldenummer: PCT/CH2003/000618
(87) Internationale Veröffentlichungsnummer: WO 2004/026401

(56) Entgegenhaltungen:
- EP-A- 0 864 337
- EP-A2- 1 396 278
- WO-A-01/00276
- JP-A- 10 094 617
- JP-A- 11 216 195

## Beschreibung

Die vorliegende Erfindung betrifft eine Anordnung zum Behandeln eines Patienten mittels Protonentherapie gemäss dem Oberbegriff von Anspruch 1 sowie Verwendungen der Anordnung.

Obwohl die Protonenbestrahlungstherapie insbesondere bei der Bestrahlung von Tumoren vorteilhaft ist, ist sie infolge hoher Kosten und raumintensiver Installationen nach wie vor nur sehr eingeschränkt verbreitet.

Die Protonentherapie wird heutzutage vorzugsweise mit Hilfe einer drehbaren Strahlführung, der sogenannten Gantry, appliziert.

Die Strahlführung ist auf einem Drehgestell montiert und kann somit um den Patiententisch gedreht werden. Mit dieser Konfiguration kann der Strahl von mehreren Einstrahlrichtungen auf den liegenden (in seiner Moulage auf dem Patiententisch immobilisierten) Patienten sequentiell appliziert werden.

Für die Therapie mit Protonen von tiefliegenden Tumoren liegt die benötigte Energie des Protonenstrahles zwischen 70 und 250 MeV. Die Strahlführung benötigt wegen der erheblichen Steifheit des Strahles viel Platz (mit Normalmagneten beträgt der minimale Ablenkradius, welcher auf dem Strahl appliziert werden kann, mindestens 1.3m) und ist entsprechend massiv. Gantries für die Protonentherapie sind typischerweise 10m lang, weisen einen Durchmesser von 4 bis zu 12m auf und wiegen bis zu hundert Tonnen.

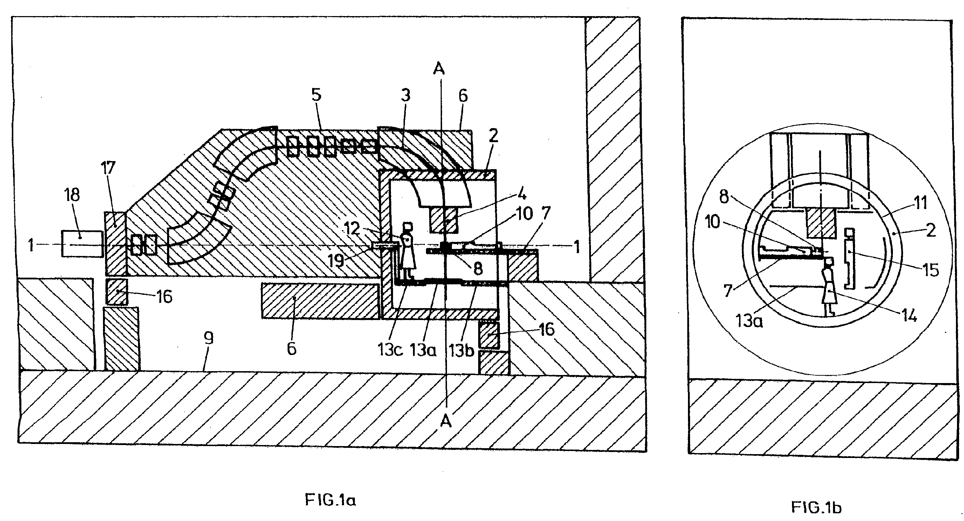

Von einer modernen Gantryanlage wird erwartet, dass der Patiententisch (zusätzlich zu der axialen Rotation der Gantry) auch um die vertikale Achse in die horizontale Ebene gedreht werden kann. Damit will man erreichen, dass von der Anlage her praktisch alle Einstrahlrichtungen auf den liegenden Patienten gewählt werden können (ein 4π Raumwinkel im Idealfall). Die Rotation des Patiententisches und der Gantry sind deshalb funktionell gleich begründet. Das neue Konzept dieser Arbeit ist, die Rotation der Gantry auf +, -135° bzw. +, -90° zu beschränken und dafür die volle Drehbarkeit bis 360° des Tisches zu nützen (wie die geographische Breiten- und Längeneinteilung in der Weltgeographie). Aus historischen Gründen ist diese Zuordnung bei allen bisherigen Systemen umgekehrt realisiert, d.h. Rotation der Gantry um 360° und des Tisches lediglich maximal um 180°. Die heutigen marküblichen Systeme basieren auf dem Konzept, dass die Gantry vollständig um 360° um die longitudinale Strahlachse gedreht werden kann. Das Prinzip von einem solchen konventionellen System ist nachfolgend schematisch und unter Bezug auf Figur 1 detailliert beschrieben.

Das Paul Scherrer Institut hat bereits Wesentliches zur Entwicklung der Protonentherapie beigetragen. Die Gantry 1 des PSI ist, nach Loma Linda in California, die zweitälteste Protonengantry der Welt. Sie ist seit 1996 in Betrieb und ist immer noch die einzige, welche mit einem dynamischen Scanning des Strahles benützt wird. Nach wie vor ist sie die Gantry mit dem kleinsten Durchmesser (4m) überhaupt. Einige abgeleitete Merkmale des PSI Systems sind in abgeänderter Form für Industriepatente bereits verwendet worden. Derartige Systeme sind beispielsweise in den beiden europäischen Patenten EP 0 911 964 und EP 0 864 337 beschrieben.

Die Erfahrung mit dem gegenwärtigen System hat uns gezeigt, wie wichtig eine gute Zugänglichkeit zum Patiententisch in jeder Phase der Bestrahlung ist.

In der internationalen Patentanmeldung WO 01/00276 zu diesem Thema aus dem Jahre 2000 (Priorität 1999) beschreibt eine Vorrichtung zum Durchführen einer Protonentherapie, wobei die Protonenstrahlführungs- und -steuerungseinrichtung lediglich um einen Winkel von maximal 270 drehbar bzw. rotierbar ausgebildet ist, so dass, wie gefordert, eine gute Zugänglichkeit zum Patiententisch in jeder Phase der Bestrahlung gewährleistet wird. Auf eine detaillierte Beschreibung der WO 01/00276 wird verzichtet, lediglich nachfolgend unter Bezug auf Figur 2 wird schematisch eine Protonenbestrahlungsanordnung analog der WO 01/00276 näher erläutert.

Obwohl in der Anordnung gemäss WO 01/00276 die Forderung nach der Zugänglichkeit zum Patiententisch erfüllt wird, bleibt die Forderung nach Dimensionsreduktion und Vereinfachung der Gantry bestehen. Es ist somit eine Aufgabe der vorliegenden Erfindung, eine weitere Anordnung für die Durchführung von Protonentherapie vorzuschlagen, welche einerseits die ständige Zugänglichkeit zum Patiententisch ohne Einbussen in der Funktionalität gewährleistet und andererseits eine weitere Vereinfachung und Erhöhung der Genauigkeit bei der Durchführung der Protonentherapie, sowie insbesondere eine weitere Reduktion der Dimensionen von Komponenten der Gantry bzw. der Gantry selbst ermöglicht.

Erfindungsgemäss wird die gestellte Aufgabe primär mittels einer Anordnung gemäss dem Wortlaut nach Anspruch 1 gelöst. Erfindungsgemäss vorgeschlagen wird, dass die Protonenstrahlführungs- und steuerungseinrichtung, nachfolgend Gantry genannt, in Richtung der Strahlführung dem Patiententisch vorgeschaltet und nachgeschaltet um eine Horizontalachse rotierbar bzw. drehbar abgestützt gelagert ist. Wesentliches Merkmal bleibt dabei, dass der Patiententisch bzw. die Betreuung eines Patienten von einer Seite her jeder Zeit zugänglich bzw. möglich ist.

Die erfindungsgemässe Anordnung bzw. die Gantry ist gemäss einer bevorzugten Ausführungsvariante über eine kleine Rolle an ihrer vordersten Stelle drehbar gelagert. Die Gantry ist dabei analog der Ausführung gemäss WO 01/00276 aus einer Horizontalebene in etwa verlaufend durch die Drehachse um je bis zu 135° nach oben und nach unten drehbar bzw. rotierbar angeordnet. Der erwähnte Winkel von insgesamt 270° kann selbstverständlich, je nach Ausführung der Gantry, auch kleiner gewählt werden, sollte aber nicht weniger als ± 90° aus der Horizontalebene bzw. insgesamt 180° betragen.

Ein Vorteil der erfindungsgemäss vorgeschlagenen Anordnung liegt darin, dass mindestens ein Teil der Verkabelung von der vorderen Seite der Gantry angeschlossen werden kann, was eine saubere, örtliche Trennung der Kabelführungen für Messsignale und Überwachungssensoren von der Magneteinspeisung erlaubt. Dadurch können allfällige Interferenzen der Signale vermieden werden, wodurch auch die Genauigkeit der Protonenstrahlführung erhöht werden kann, indem die empfindlichen Messeinrichtungen von den Magnetspeisungen nicht gestört werden.

Gemäss einer weiteren Ausführungsvariante wird der massive Teil der Protonenstrahlführungs- und steuerungseinheit bzw. der Gantry, wie beispielsweise der 90°-Magnet verdeckt, so dass für den Patienten nur die Nozzle des Protonenstrahls sichtbar wird. Mit "Nozzle" (Nase) bezeichnet man das Gehäuse und die darin enthaltene Bestrahlungsapparatur am Strahlaustritt der Gantry unmittelbar vor dem Patienten. Dort befinden sich z.B. die Messsysteme für das Monitorieren des Strahles (Messung der Position und Intensität des Strahles), der Reichweitenmodulator, Laser und Fernsehkameras für die Positionierung des Patienten und die Einstellung des Strahles, usw. Dieser Teil ist für den Patienten sichtbar. Dagegen der massive Teil mit dem 90°-Magneten, welcher beispielsweise ca. 20 t wiegen kann, Ausdehnungen von 2 m Höhe und 1 m Breite oder mehr aufweisen kann, kann bei der vorliegenden Anordnung gut verdeckt werden, indem die Bestrahlungskammer als ein halboffener Raum, beispielsweise in Zimmergrösse, ausgestaltet ist, wobei lediglich von der Seite der Zugänglichkeit her die Seitenwand fehlt.

Die wesentlichen Vorteile der erfindungsgemäss vorgeschlagenen Anordnung sind die folgenden:
- Die Gestaltung der Bestrahlungskammer ist einfacher;
- Die Kosten und der Aufwand für die Ausgestaltung eines beweglichen Hilfsbodens entfallen;
- Der Patiententisch ist jederzeit zugänglich;
- Kleinere Dimensionierung der Gantry möglich, bzw. die Grube unter dem Patiententisch ist weniger tief auszubilden.
- Einfacheres und billigeres System;
- Höhere Zuverlässigkeit und Genauigkeit;
- Durch Anbringen der frontseitigen Rolle kleinerer Durchmesser der Anlage bzw. der Rollvorrichtung
- Die neue Anordnung ist nicht nur für zentrische Systeme, wie in der WO 01/00276 beschrieben, geeignet, sondern auch für exzentrische Systeme (Gantry) wie die gegenwärtige Anlage (Gantry) des Paul Scherrer Instituts (PSI).

Weitere bevorzugte Ausführungsvarianten der erfindungsgemässen Anordnung sind in den abhängigen Ansprüchen charakterisiert.

Die erfindungsgemässe Anordnung eignet sich insbesondere zum Behandeln eines Patienten mittels Protonentherapie, wobei eine auf dem Patiententisch liegende Person durch Bewegen von Patiententisch und Protonenstrahlführung (Gantry) derart positioniert wird, dass der Protonenstrahl die zu behandelnde Stelle im Körper des Patienten aus jeder gewünschten Richtung bestrahlen kann und dass der Patiententisch jederzeit von einer Seite her zugänglich bleibt.

Die Erfindung wird nun anschliessend beispielsweise und unter Bezug auf die beigefügten Figuren näher erläutert.

Dabei zeigen:
- Fig. 1a und 1b: schematisch in Seitenansicht und im Querschnitt entlang der Linie A-A eine marktübliche, kompakte Protonen-Gantry mit konventioneller Mechanik,
- Fig. 2a und 2b: schematisch in Seitenlängsansicht und im Schnitt entlang der Linie B-B eine kompakte Protonen-Gantry entsprechend der WO 01/00276,
- Fig. 3a und 3b: schematisch in Seitenlängsansicht und im Schnitt entlang der Linie C-C eine erfindungsgemässe Protonen-Gantryanordnung, frontseitig abgestützt,
- Fig. 4: eine erfindungsgemässe zentrische Gantry in seitlicher Perspektive,
- Fig. 5: eine erfindungsgemässe exzentrische Gantry in seitlicher Perspektive, und
- Fig. 6a und 6b: schematisch in Seitenlängsansicht und im Schnitt entlang der Linien C-C und D-D eine weitere Ausführungsvariante einer erfindungsgemässen Protonen-Gantryanordnung.

Für das bessere Verständnis der vorliegenden Erfindung wird nun zunächst anhand der Figuren 1a und 1b schematisch ein heute marktübliches System beschrieben, basierend auf dem Konzept, dass die Protonenstrahlanlage bzw. die Gantry vollständig um 360° um die longitudinale Strahlachse gedreht werden kann.

Ein Patient 10 wird in einer zylindrischen Kammer, der Bestrahlungskammer 2, behandelt. Die Strahlführung 3 wird ausserhalb der Kammer geführt. Nur der letzte Teil, das sogenannte Nozzle 4, mit den Messeinrichtungen für die Kontrolle der räumlichen Verteilung der Dosis, ragt in die Kammer hinein (der Patient muss möglichst nahe am Nozzle platziert werden können).

Das Ganze wird durch ein Drehgestell 5 getragen, inklusive das Gegengewicht 6 der Strahlführung. Position 16 zeigt die Lage der Räder 16, worauf über zwei Rollen sich die Gantry dreht. Die hintere Rolle 17 kann mit einem Radius von ca. 1m relativ klein gehalten werden. Die vordere Rolle ist die Bestrahlungskammer selbst. Position 18 zeigt die Kabeltrommel, wo die Verkabelung der Gantry angeschlossen wird.

Der Patiententisch 7 wird benützt, um den Patienten im Isozentrum 8 der Maschine zu positionieren (Isozentrum = Treffpunkt des Strahles bei der Rotation der Gantry). Weil Bestrahlungskammer und Nozzle mit der Gantry mitdrehen, ist der Tisch direkt vor der Kammer verankert. Die Tischplatte mit dem darauf liegenden Patienten ragt von aussen in die Kammer hinein.

Die medizinische Forderung, dass der Patiententisch in der horizontalen Ebene um den Tumor herum gedreht werden kann (im Isozentrum der Maschine - um die Achse A-A gemäss Fig. 1a) bedingt, dass der innere Radius 11 der zylindrischen Bestrahlungskammer mindestens 2m aufweisen soll (Länge des menschlichen Körpers). Um die Drehung und den dazu notwendigen Platzbedarf bei Kopfbestrahlung zu verdeutlichen, wurde der Patient 10 in Figur 1 in der Front- und Seitenansicht jeweils senkrecht zum Beobachtungspunkt gezeichnet.

Die Höhe der Patiententischplatte 7 über dem Boden der Bestrahlungskammer ist somit auch auf ca. 2m festgelegt. Der Zugang des Personals 12 zum Patienten in Bestrahlungsposition ist so, ohne zusätzliche Vorrichtungen, nicht zufriedenstellen. Die Notwendigkeit eines flachen falschen Bodens 13 im Inneren der Kammer wird in der Frontsicht der Figur 1 verdeutlicht, wo die Lage des Hilfspersonals mit 15 und ohne 14 eines solchen Hilfsbodens dargestellt wird.

Die Konstruktion eines flachen Bodens 13 im Innern der zylindrischen Bestrahlungskammer ist aus verschiedenen Gründen ziemlich kompliziert.

Bei der Applikation mit Strahl von unten, muss der falsche Boden sich öffnen und sich an die Rotation der Nozzle anpassen (beweglicher Teil der Abdeckung für die Nozzle 13a). Auch die übrigen Teile des Bodens, Teil hinten 13c und Teil vorne 13b können nicht direkt in die Kammer verankert werden, weil die Bestrahlungskammer sich mit der Gantry dreht, der Boden aber horizontal bleiben soll.

Der vordere Teil 13b des Bodens kann von ausserhalb der Kammer gestützt werden. Das grösste Problem liegt aber im hinteren Teil der Kammer, im Teil 13c, welcher durch die Bewegung der Nozzle "weggeschnitten" wird. Das Problem kann hier mit Hilfe einer eingebauten Gegendrehung 19 zu der Drehung der Gantry im hinteren Teil der Kammer gelöst werden, nach dem benützten Prinzip für den Einbau des Patiententisches in die Gantry 1 des PSI.

Diese Idee der (Gegendrehung) wurde nachträglich für die Stütze des falschen Bodens übernommen. Die heutzutage existierenden kommerziellen Anlagen haben alle, entweder keinen Boden in der zylindrischen Kammer, oder einen Boden, welcher nach dem oben geschilderten Prinzip (hintere Stütze mit Gegendrehung, vordere Stütze fest) aufgebaut ist.

Ein beweglicher Boden im Innern der zylindrischen Bestrahlungskammer ist zwar machbar, aber bestimmt nicht billig und einfach.

Ziel der WO 01/00276 des PSI war eine bessere Lösung zu bieten, welche eine bessere Zugänglichkeit zum Patiententisch, ohne Einbussen in der Funktionalität, gewährleistet und zwar mit dem Boden 13b und 13c fest installiert ohne Gegendrehung.

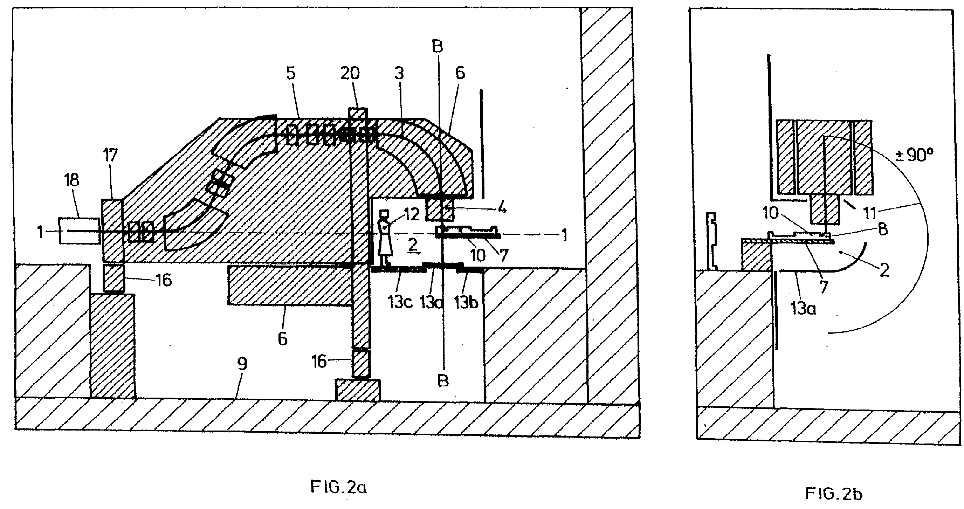

Die Figuren 2a und 2b zeigen schematisch die in der WO 01/00276 vorgeschlagene Anordnung der Gantry im Längsschnitt und im Schnitt entlang der Linie B-B. In den Figuren 2a und 2b ist zu beachten, dass die Bezeichnung derselben Teile entsprechend den Figuren 1a und 1b dieselben Bezugszeichen verwendet werden.

Der Unterschied der Protonenbestrahlungsanlage bzw. der Gantry, beschrieben in den Figuren 2a und 2b zur entsprechenden Anlage, dargestellt in den Figuren 1a und 1b, liegt primär darin, dass die Drehung der Gantry auf ± 90° (+180, -120°) in der Vertikalen auf nur einer Seite der Gantry limitiert ist. Dies wird insbesondere aus der Darstellung gemäss Figur 2b deutlich sichtbar. Die nicht einstellbaren Winkel der Gantry werden spiegelsymmetrisch gewählt und mit Hilfe einer Drehung des Patiententisches um 180° in der horizontalen Ebene erreicht. Bei voller Verfügbarkeit der Drehung des Tisches in die Horizontale sind mindestens theoretisch keine Einschränkungen in der Wahl der Einstrahlrichtung vom Prinzip her zu erwarten. Der gewonnene Platz auf der gegenüberliegenden Seite der Strahlführung kann somit für die feste Installation des Patiententisches 7 und des festen Bodens 13 im Loch der Grube benützt werden. Ebenfalls aus den Figuren 2a und 2b deutlich erkennbar wird, dass die Zugänglichkeit zum Patiententisch wesentlich einfacher und besser gewährleistet ist, als vergleichsweise bei der Anordnung gemäss den Figuren 1a und 1b.

Wie insbesondere aber auch aus Figur 2a deutlich erkennbar ist, ist die Anlage aufgrund der Verwendung der grossen Drehscheibe 20 sehr gross und benötigt einen grossen Platzbedarf. Eine ca. 7 m grosse Drehscheibe ist schwierig zu bauen und zu transportieren. Die verlangte Präzision des Isozentrums von besser als 1 mm ist entsprechend schwierig zu realisieren. Zudem ist zu beachten, dass die Räder 16 in Figur 2 unter der grossen Rolle 20 liegen und somit die Grube deshalb tiefer ausgebildet ist als diejenige dargestellt in den Fig. 1a und 1b.

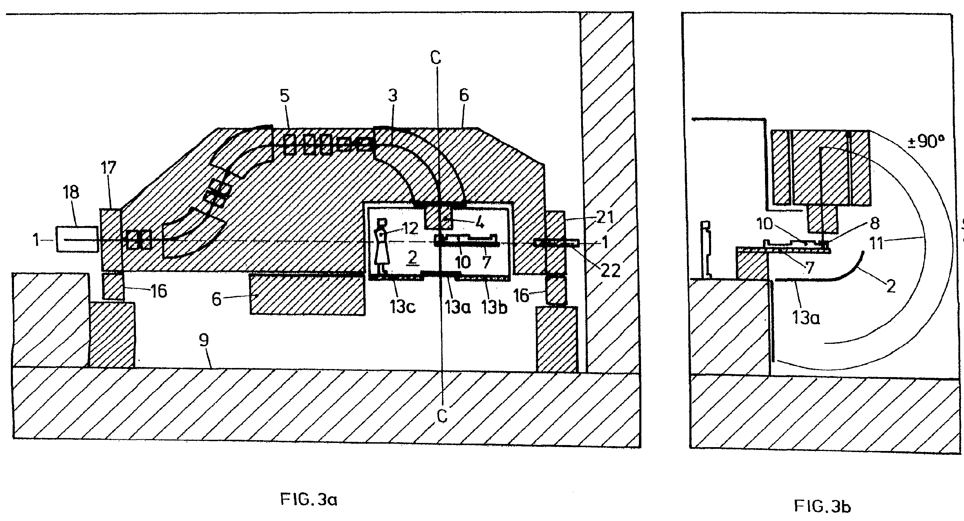

Um die Anlage weiter zu vereinfachen, wurde nun die in den Figuren 3a und 3b schematisch dargestellte erfindungsgemässe Anordnung gewählt. Die Grundidee der neu entwickelten Gantry besteht darin, die Bestrahlungseinheit bzw. die Gantry erst nach der Bestrahlungskammer mit einer kleinen Rolle 21 zu stützen. Dadurch entfällt die Notwendigkeit der in Figur 2a dargestellten Drehscheibe mit dem entsprechend grossen Durchmesser. Die übrigen Merkmale der Anordnung gemäss Figuren 2a und 2b werden im neuen Konzept im Wesentlichen beibehalten. Figur 3a zeigt die erfindungsgemässe Anordnung schematisch in Längsseitenansicht und Figur 3b im Querschnitt entlang der Line C-C. Dabei wird nun deutlich erkennbar, dass die Strahlführung 3 analog der Anordnung gemäss den Figuren 2a und 2b seitlich begrenzt drehbar ist. Wiederum ist die Bestrahlungskammer 2 von einer Seite der Gantry her zugänglich. Die Bestrahlungskammer 2 ist ein halboffener Raum in Zimmergrösse mit beispielsweise ca. 4,5m in Längsrichtung, ca. 4m in der Tiefe von der Seite und ca. 2,2 bis 2,5m in der Höhe. Für den Patienten ist nur das Protonenstrahlaustrittsfenster bzw. die Nozzle sichtbar, der massive Teil der Gantry, wie z.B. der 90°-Magnet ist verdeckt. Der Boden ist in den Bereichen 13c und 13b analog den Figuren 2a und 2b fest installiert mit Ausnahme eines Schlitzes (ca. 50cm breit) für die Führung der Nozzle. Dieser Bereich kann beispielsweise mit einer beidseitig geführten Rollabdeckung zugedeckt sein (13a). Wie bereits oben erwähnt, liegen die klaren Vorteile der erfindungsgemässen Anordnung in den folgenden Merkmalen:
- Der wohl grösste Vorteil dieser Anordnung liegt darin, dass durch die Lagerung der Gantry über eine kleine Rolle an ihrer vordersten Stelle und der damit verbundenen Drehung die Herstellkosten der grossen Rolle 20 aus Fig. 2 entfallen.
- Im Weiteren besteht nach wie vor der Vorteil dieser Lösung in der guten Zugänglichkeit zum Patiententisch mit einem fast ausschliesslich festen Boden.
- Der Platz um den Patiententisch ist gross und kann komfortabel ausgestaltet werden, wobei sich auch die Möglichkeit bietet weitere Geräte auf dem festen Boden in Reichweite des Tisches zu installieren, wie z.B. einen Tisch für Narkose, einen Computertomographen (CT), einen Positronemissionstomographen (PET), etc..
- Mindestens ein Teil der Verkabelung kann an der vorderen Seite der Gantry angeschlossen werden. Dies erlaubt eine saubere örtliche Trennung der Kabelführungen für die Messsignale bzw. Überwachungssensoren von der Magnetspeisung.
- Die Anordnung erlaubt eine platzsparende Gestaltung des Zugangslabyrinthes bzw. die Gestaltung der Abschirmung.
- Die Abschirmung an den Wänden der Bestrahlungskammer kann reduziert werden, wo diese von der Strahlführung immer abgewandt sind.

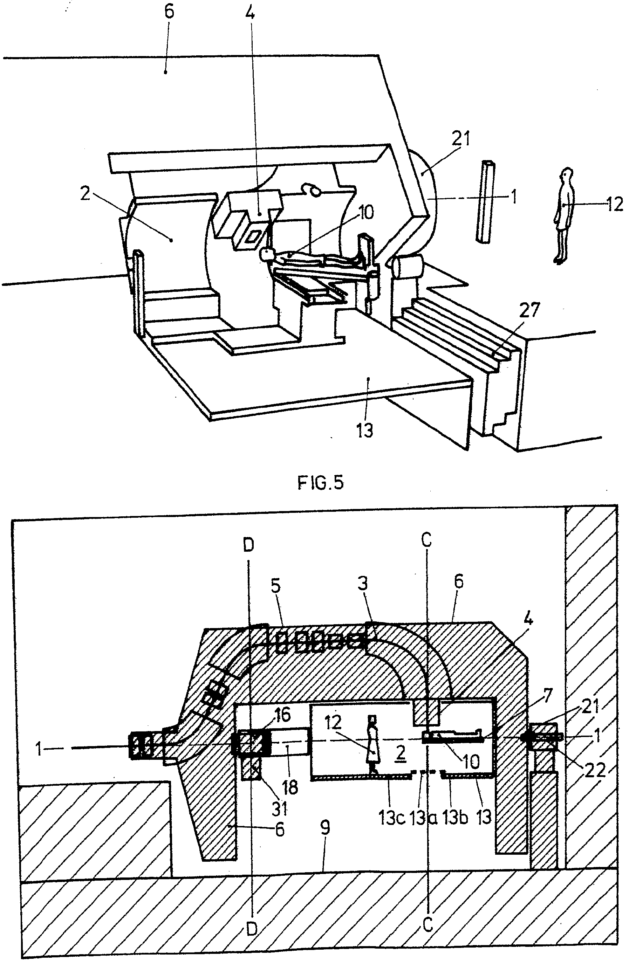

Ein weiterer Vorteil des neuen Konzeptes liegt darin, dass es nebst zentrischen kompakten Systemen, wie dargestellt in den Figuren 2a und 2b auch für exzentrische kompakte Systeme anwendbar ist, wie beispielsweise in Figur 5 dargestellt. Wegen der Exzentrizität muss jedoch die Bestrahlungskammer an der Gantry, beispielsweise im Sinne eines Gegengewichtes aufgehängt werden, und mit Hilfe der Gegendrehung horizontal gehalten werden.

Zudem ist die neue Lösung vom Konzept her allgemein flexibler als die Lösung gemäss den Figuren 2a und 2b. Währenddem letztere nur für eine kompaktere Gantry möglich ist, kann das neue System sowohl für kompakte, kompaktexzentrische als auch für "long throw Gantries" sowie für Strahlapplikation mit Scanning oder Scattering Technologie verwendet werden. Die grosse Variabilität ist attraktiv für eine kommerzielle Nutzung, besonderes wenn kundenorientierte Lösungen anzubieten sind.

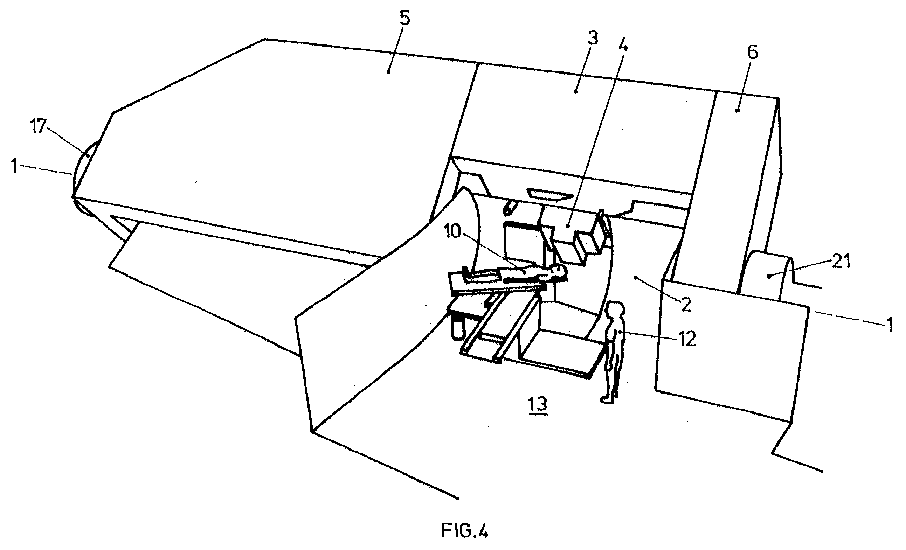

Unter Bezug auf die Figuren 4 und 5 ist anhand von perspektivischen Ansichten die Verwendung der erfindungsgemässen Anordnung, sowohl in einem kompakten zentrischen System dargestellt, wie auch bei einem exzentrischen, kompakten Gantry-System.

Figur 4 zeigt in Perspektive eine zentrische Gantry, welche um die Drehachse 1 um die beiden Rollen 17 und 21 drehbar gelagert ist. Die Bestrahlung erfolgt dabei von oben rechts (vom Patienten aus gesehen).

Zentrisch heisst die Anordnung deshalb, da die Gantry um die starr bzw. fest angeordnete Bestrahlungskammer 2 herum rotierbar ist, womit der Kammerboden 13 fest angeordnet ist.

Demgegenüber ist in der Anordnung gemäss Figur 5 die Bestrahlungskammer exzentrisch in einer Kreisbewegung um die Drehachse 1 herum drehbar, womit selbstverständlich auch der Patiententisch 10 eine entsprechende Bewegung, je nach Lage der Bestrahlung, durchzuführen hat. Hier erfolgt die Bestrahlung von oben links. Um jederzeit eine Zugänglichkeit des Kammerbodens 13 für eine Pflegeperson 12 zu gewährleisten, ist ein beweglicher, beispielsweise treppenartiger Zugang 27 notwendig. Erfolgt beispielsweise die Bestrahlung in horizontaler Richtung, entfällt die Notwendigkeit der Treppe 27, indem der Kammerboden 13 niveaugleich ist zur Umgebung. Erfolgt jedoch die Bestrahlung von unten, wird entsprechend der Kammerboden 13 nach oben angehoben und liegt oberhalb des Umgebungsniveaus.

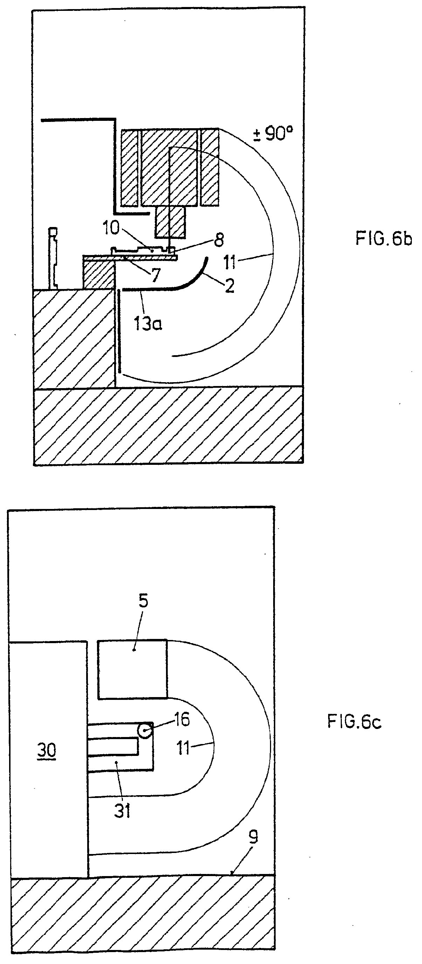

In den Figuren 6a und 6b ist eine weitere Ausführungsvariante einer erfindungsgemässen GantryAnordnung bzw. -Anlage dargestellt. Die in den Figuren 6a und 6b dargestellte Anlage unterscheidet sich von derjenigen, dargestellt in den Figuren 3a und 3b, darin, dass die hintere Lagerung der Gantry nicht endständig angeordnet ist, sondern gegen den Innenraum bzw. gegen den Bestrahlungsraum 2 hin gerichtet. Im Übrigen entspricht die Gantry von Figur 6a derjenigen aus Figur 3a, wie auch der Schnitt entlang der Linie C-C, dargestellt in Fig. 6b. Hingegen zeigt Figur 6c den Schnitt entlang der Linie D-D, d.h. die hintere Lagerung der Gantry. Dabei ist das hintere Drehrad 16 seitlich an der hinteren Kammerwandung 30 über einen beispielsweise U-förmigen Bügel 31 abgestützt, d.h. die Abstützung erfolgt nicht wie in Figur 3a dargestellt auf dem Kammerboden 9. Entsprechend ist es möglich, die Kabeltrommel 18 im mittleren Bereich der Gantry zu realisieren. Durch die Lagerung der Gantry, wie dargestellt in den Figuren 6a bis 6c wird das Gestell kürzer und durch die kürzere Distanz zwischen hinterer und vorderer Rolle 16 erreicht man eine bessere Stabilität des Gestells, d.h. es besteht weniger Gefahr des Durchhängens. Die übrigen Vorteile, wie beschrieben unter Bezug auf die Figuren 3a und 3b bleiben selbstverständlich in der Anordnung gemäss Figuren 6a bis 6c erhalten.

Bei den Darstellungen in den Figuren 1 bis 6 handelt es sich selbstverständlich nur um Beispiele die dazu dienen, die vorliegende Erfindung näher zu erläutern. Selbstverständlich ist es möglich, die erfindungsgemäss dargestellte Protonenbestrahlungs-Anordnung auf x-beliebige Art und Weise abzuändern, zu modifizieren oder durch weitere Elemente zu ergänzen. Hierzu verwiesen sei insbesondere auf die WO 01/00276, deren speziellen Ausführungsvarianten ebenfalls in Kombination mit den erfindungsgemäss definierten Merkmalen benutzt werden können.

## Patentansprüche

1. Anordnung zum Behandeln eines Patienten mittels Protonentherapie, umfassend eine Gantry (3) mit Magneten und Quadrupolen und einem Austrittsfenster (4), um den Protonenstrahl an die zu behandelnde Stelle im Patienten zu führen bzw. zu richten, sowie einen steuerbar bewegbaren Patiententisch (7, 10), um den Patienten in eine gewünschte Position in Bezug auf den Protonenstrahl zu bewegen, wobei durch die Lagerung der Gantry (3) eine Horizontaldrehachse definiert wird und das Isozentrum der Gantry einem Schnittpunkt des Protonenstrahls mit der Horizontaldrehache entspricht, wobei:
a) der Patiententisch (7, 10) in einer horizontal verlaufenden Ebene angeordnet ist und in der horizontal verlaufenden Ebene um mindestens 180° im Isozentrum der Gantry (3) drehbar gelagert ist,
b) die Gantry (3) aus der horizontal verlaufenden Ebene um höchstens -135° bis höchstens +135° auf der einen Seite des Patiententisches (7, 10) um die Horizontaldrehachse drehbar angeordnet ist, so dass der Patiententisch (7, 10) von der anderen Seite her jederzeit zugänglich bleibt,
c) die Gantry in Richtung der Horizontaldrehachse dem Patiententisch vorgeschaltet und nachgeschaltet um die Horizontaldrehachse drehbar abgestützt gelagert ist; und
d) eine Bestrahlungskammer (2) als halboffener, von mindestens einer Seite der Gantry (3) permanent zugänglicher Raum ausgestaltet ist, welcher einen fest installierten Bodenbereich (13, 13b, 13c) und im Bereich für die Führung des Austrittsfensters (4), das in die Bestrahlungskammer (2) hineinragt, einen Schlitz (13a) aufweist, wobei der Patiententisch (7, 10) in der Bestrahlungskammer (2) angeordnet ist.

2. Gantry nach Anspruch 1,
**dadurch gekennzeichnet, dass**
der Schlitz (13a) mit einer beidseitig geführten Rollabdeckung zudeckbar ist.

3. Gantry nach Anspruch 1 oder 2,
dadurch gekennzeichet, dass
die drehbar abgestützte Lagerung der Gantry mittels eines Rollenpaares (17, 21) erzielt ist, wobei sich jede Rolle (17, 21) auf jeweils einem Rad (16) abstützt.

## Claims

1. System for treating a patient by means of proton therapy, comprising a gantry (3) with magnets and quadripoles and an exit port (4) to lead, respectively point, the proton beam to the spot in the patient that has to be treated, as well as a controllably movable patient table (7, 10) in order to position the patient in a desired position with regards to the proton beam, with a horizontal rotation axis being defined by means of the position of the gantry (3) and the isocenter of the gantry corresponding to the intersection of the proton beam and the horizontal rotation axis, whereby:
a) the patient table (7, 10) is arranged in a horizontally running plane and is rotatable mounted by at least 180° in the isocenter of the gantry (3) in the horizontally running plane,
b) the gantry (3) from the horizontally running plane is rotatable mounted around the horizontal rotation axis at most -135° to +135° at most on one side of the patient table (7, 10) in order that the patient table (7, 10) remains always accessible from the other side,
c) the gantry is upstream the patient table in the direction of the horizontal rotation axis and is mounted rotatable and supported downstream around the horizontal rotation axis; and
d) a radiation chamber (2) is embodied as a half open room, permanently accessible from at least one side of the gantry (3) and comprises a firmly installed ground area (13, 13b, 13c) and further a slot (13a) within the range for the conduction of the exit port (4), that projects into the radiation chamber (2), whereby the patient table (7, 10) is arranged in the radiation chamber (2).

2. A gantry according to claim 1,
**characterized in that**
the slot (13a) is coverable with a bilaterally conducted roll covering.

3. A gantry according to claim 1 or 2,
**characterized in that**
the rotatable supported bearing of the gantry is achieved by means of a roll pair (17, 20), with each roll (17, 21) supporting itself in each case on a wheel (16).

## Revendications

1. Installation pour traiter un patient par protonthérapie, comprenant un portique ou gantry (3) muni d'aimants et de quadrupôles et d'une fenêtre de sortie (4) servant à guider resp. à diriger le faisceau de protons sur la zone à traiter du patient, et une table de patient (7, 10) à déplacement commandable, servant à déplacer le patient vers une position souhaitée par rapport au faisceau de protons, le montage du portique (3) définissant un axe de rotation horizontal et l'isocentre du portique correspondant à un point d'intersection du faisceau de protons avec l'axe de rotation horizontal, installation dans laquelle :
a) la table de patient (7, 10) est disposée dans un plan horizontal et est montée dans le plan horizontal de manière à pouvoir tourner de 180° dans l'isocentre du portique (3),
b) le portique (3) est disposé dans le plan horizontal de manière à pouvoir tourner de -135° au maximum jusqu'à +135° au maximum autour de l'axe de rotation horizontal sur un côté de la table de patient (7, 10), de sorte que la table de patient (7, 10) reste accessible à tout moment depuis l'autre côté,
c) le portique est monté d'une part dans le sens de l'axe de rotation horizontal de la table de patient, et d'autre part en appui rotatif autour de l'axe de rotation horizontal ; et
d) une chambre d'irradiation (2) est configurée comme espace semi-ouvert et accessible à tout moment depuis au moins un côté du portique (3), lequel espace présente une partie de fond (13, 13b, 13c) montée de manière fixe et, dans la partie réservée au guidage de la fenêtre de sortie (4) qui s'étend dans la chambre d'irradiation (2), une fente (13a), la table de patient (7, 10) étant disposée dans la chambre d'irradiation (2).

2. Portique selon la revendication 1,
**caractérisé en ce que**
la fente (13a) peut être couverte par un recouvrement roulant guidé des deux côtés.

3. Portique selon la revendication 1 ou 2,
**caractérisé en ce que**
le montage en appui rotatif du portique est obtenu au moyen d'une paire de rouleaux (17, 21), chaque rouleau (17, 21) s'appuyant sur une roue (16).
